# EUROPEAN PATENT SPECIFICATION

(11) **EP 3 097 802 B1**
(45) Date of publication and mention of the grant of the patent: **26.06.2024**
(21) Application number: 15169205.0
(22) Date of filing: 26.05.2015
(51) Int. Cl.: A24F 7/04, A24F 40/40, A24F 40/485, A24F 40/10

(54) **MOUTHPIECE FOR AN ELECTRONIC SMOKING DEVICE**
MUNDSTÜCK FÜR EINE ELEKTRONISCHE RAUCHVORRICHTUNG
EMBOUT POUR DISPOSITIF À FUMER ÉLECTRONIQUE

(43) Date of publication of application: 30.11.2016
(73) Proprietor: Fontem Ventures B.V., 1043 NT Amsterdam (NL)
(72) Inventor: Biel, Stefan, 22761 Hamburg (DE); Borkovec, Vaclav, 22761 Hamburg (DE); Yener, Emin, 22761 Hamburg (DE); Chaves, Liliana, 22761 Hamburg (DE)
(74) Representative: Gulde & Partner

(56) References cited:
- CN-U- 203 646 511
- CN-U- 203 748 667
- US-A- 5 878 752
- US-A1- 2013 192 615
- US-A1- 2014 338 680
- US-A1- 2014 366 898
- US-A1- 2015 196 058

## Description

### FIELD OF INVENTION

The present invention relates generally to electronic smoking devices and in particular electronic cigarettes.

### BACKGROUND OF THE INVENTION

An electronic smoking device, such as an electronic cigarette (e-cigarette), typically has a housing accommodating an electric power source (e.g. a single use or rechargeable battery, electrical plug, or other power source), and an electrically operable atomizer. The atomizer vaporizes or atomizes liquid supplied from a reservoir and provides vaporized or atomized liquid as an aerosol. Control electronics control the activation of the atomizer. In some electronic cigarettes, an airflow sensor is provided within the electronic smoking device which detects a user puffing on the device (e.g., by sensing an under-pressure or an air flow pattern through the device). The airflow sensor indicates or signals the puff to the control electronics to power up the device and generate vapor. In other e-cigarettes, a switch is used to power up the e-cigarette to generate a puff of vapor.

In some electronic smoking devices the vaporized or atomized liquid is provided as aerosol in a central passage between the atomizer to an air inhalation port where the user puffs on the device thereby generating the flow of gas.

The document CN 203/748 667 U discloses all the preamble of independent claim 1. The documents CN 203/646 511 U, US 2014/366898 A1 and US2015/196 058 A1 are particularly interesting examples of the prior art.

### SUMMARY OF THE INVENTION

In accordance with one aspect of the present invention there is provided a mouthpiece for an electronic smoking device according to claim 1 and an electronic smoking device comprising the mouthpiece according to claim 5.

The characteristics, features and advantages of this invention and the manner in which they are obtained as described above, will become more apparent and be more clearly understood in connection with the following description of exemplary embodiments, which are explained with reference to the accompanying drawings.

### BRIEF DESCRIPTION OF THE DRAWINGS

In the drawings, same element numbers indicate same elements in each of the views:
Figure 1 is a schematic cross-sectional illustration of an exemplary e-cigarette having a mouthpiece according to an embodiment;
Figure 2 is a schematic cross-sectional illustration of a an exemplary e-cigarette having a mouthpiece according to another embodiment;
Figure 3 is a schematic cross-sectional illustration of a mouthpiece according to yet another embodiment; and
Figure 4 is a schematic cross-sectional illustration of a mouthpiece according to even yet another embodiment.

### DESCRIPTION OF THE PREFERRED EMBODIMENTS

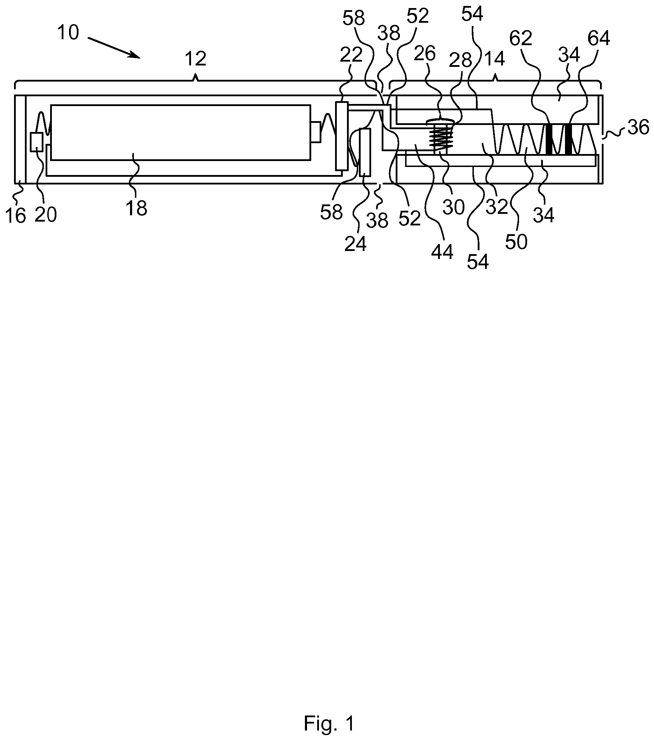

Throughout the following, an electronic smoking device will be exemplarily described with reference to an e-cigarette. As is shown in Figure 1, an e-cigarette 10 typically has an elongated housing comprising a cylindrical hollow tube having an end cap 16. The cylindrical hollow tube may be a single piece or a multiple piece tube. In Figure 1, the cylindrical hollow tube is shown as a two piece structure having a battery portion 12 and an atomizer/liquid reservoir portion 14. Together the battery portion 12 and the atomizer/liquid reservoir portion 14 form a cylindrical tube which is approximately the same size and shape as a conventional cigarette, typically about 100 mm with a 7.5 mm diameter, although lengths may range from 70 to 150 or 180 mm, and diameters from 5 to 20 mm.

The battery portion 12 and atomizer/liquid reservoir portion 14 are typically made of steel or hardwearing plastic and act together with the end cap 16 to provide a housing to contain the components of the e-cigarette 10. The battery portion 12 and an atomizer/liquid reservoir portion 14 may be configured to fit together by a friction push fit, a snap fit, or a bayonet attachment, magnetic fit, or screw threads. The end cap 16 is provided at the front end of the battery portion 12. The end cap 16 may be made from translucent plastic or other translucent material to allow an LED 20 positioned near the end cap to emit light through the end cap. The end cap can be made of metal or other materials that do not allow light to pass.

An air inlet may be provided in the end cap, at the edge of the inlet next to the cylindrical hollow tube, anywhere along the length of the cylindrical hollow tube, or at the connection of the battery portion 12 and the atomizer/liquid reservoir portion 14. Figure 1 shows a pair of air inlets 38 provided at the intersection between the battery portion 12 and the atomizer/liquid reservoir portion 14.

A battery 18, a light emitting diode (LED) 20, control electronics 22 and optionally an airflow sensor 24 are provided within the cylindrical hollow tube battery portion 12. The battery 18 is electrically connected to the control electronics 22, which are electrically connected to the LED 20 and the airflow sensor 24. In this example the LED 20 is at the front end of the battery portion 12, adjacent to the end cap 16 and the control electronics 22 and airflow sensor 24 are provided in the central cavity at the other end of the battery 18 adjacent the atomizer/liquid reservoir portion 14.

The airflow sensor 24 acts as a puff detector, detecting a user puffing or sucking on the atomizer/liquid reservoir portion 14 of the e-cigarette 10. The airflow sensor 24 can be any suitable sensor for detecting changes in airflow or air pressure such as a microphone switch including a deformable membrane which is caused to move by variations in air pressure. Alternatively the sensor may be a Hall element or an electro-mechanical sensor.

The control electronics 22 are also connected to an atomizer 26. In the example shown, the atomizer 26 includes a heating coil 28 which is wrapped around a wick 30 extending across a central passage 32 of the atomizer/liquid reservoir portion 14, the central passage being in communication with ports at opposite ends of an elongated housing of the atomizer/liquid reservoir portion 14. The coil 28 may be positioned anywhere in the atomizer 26 and may be transverse or parallel to the liquid reservoir 34. The wick 30 and heating coil 28 do not completely block the central passage 32. Rather an air gap is provided on either side of the heating coil 28 enabling air to flow past the heating coil 28 and the wick 30. The atomizer may alternatively use other forms of tempering elements, such as ceramic heaters, or fiber or mesh material heaters. Nonresistance tempering elements such as sonic, piezo and jet spray may also be used in the atomizer in place of the heating coil.

The central passage 32 is surrounded by a cylindrical liquid reservoir 34 with the ends of the wick 30 abutting or extending into the liquid reservoir 34. The wick 30 may be a porous material such as a bundle of fiberglass fibers, with liquid in the liquid reservoir 34 drawn by capillary action from the ends of the wick 30 towards the central portion of the wick 30 encircled by the heating coil 28.

The liquid reservoir 34 may alternatively include wadding soaked in liquid which encircles the central passage 32 with the ends of the wick 30 abutting the wadding. In other embodiments the liquid reservoir 34 may comprise a toroidal cavity arranged to be filled with liquid and with the ends of the wick 30 extending into the toroidal cavity.

One of the ports is provided at an end of the housing for being fitted together with the battery portion 12. The other port is an air inhalation port 36 provided at the back end of the atomizer/liquid reservoir portion 14 remote from the end cap 16. The air inhalation port 36 may be formed from the cylindrical hollow tube atomizer/liquid reservoir portion 14 or may be formed in an end cap.

In use, a user sucks on the e-cigarette 10. This causes air to be drawn into the e-cigarette 10 via one or more air inlets, such as air inlets 38 and to be drawn through the central passage 32 towards the air inhalation port 36. The change in air pressure which arises is detected by the airflow sensor 24 which generates an electrical signal that is passed to the control electronics 22. In response to the signal, the control electronics 22 activate the heating coil 28 which causes liquid present in the wick 30 to be vaporized creating an aerosol (which may comprise gaseous and liquid components) within the central passage 32. As the user continues to suck on the e-cigarette 10, this aerosol is drawn through the central passage 32 and inhaled by the user. At the same time the control electronics 22 also activate the LED 20 causing the LED 20 to light up which is visible via the translucent end cap 16 mimicking the appearance of a glowing ember at the end of a conventional cigarette. As liquid present in the wick 30 is converted into an aerosol more liquid is drawn into the wick 30 from the liquid reservoir 34 by capillary action and thus is available to be converted into an aerosol through subsequent activation of the heating coil 28.

Some e-cigarettes are intended to be disposable and the electric power in the battery 18 is intended to be sufficient to vaporize the liquid contained within the liquid reservoir 34 after which the e-cigarette 10 is thrown away. In other embodiments the battery 18 is rechargeable and the liquid reservoir 34 is refillable. In the cases where the liquid reservoir 34 is a toroidal cavity, this may be achieved by refilling the liquid reservoir 34 via a refill port. In other embodiments the atomizer/liquid reservoir portion 14 of the e-cigarette 10 is detachable from the battery portion 12 and a new atomizer/liquid reservoir portion 14 can be fitted with a new liquid reservoir 34 thereby replenishing the supply of liquid. In some cases, replacing the liquid reservoir 34 may involve replacement of the heating coil 28 and the wick 30 along with the replacement of the liquid reservoir 34. A replaceable unit comprising the atomizer 26 and the liquid reservoir 34 is called a cartomizer.

The new liquid reservoir 34 may be in the form of a cartridge having a central passage 32 through which a user inhales aerosol. In other embodiments, aerosol may flow around the exterior of the cartridge 32 to an air inhalation port 36.

Of course, in addition to the above description of the structure and function of a typical e-cigarette 10, variations also exist. For example, the LED 20 may be omitted. The airflow sensor 24 may be placed adjacent to the end cap 16 rather than in the middle of the e-cigarette. The airflow sensor 24 may be replaced with a switch which enables a user to activate the e-cigarette manually rather than in response to the detection of a change in air flow or air pressure.

Different types of atomizers may be used. Thus for example, the atomizer may have a heating coil in a cavity in the interior of a porous body soaked in liquid. In this design aerosol is generated by evaporating the liquid within the porous body either by activation of the coil heating the porous body or alternatively by the heated air passing over or through the porous body. Alternatively the atomizer may use a piezoelectric atomizer to create an aerosol either in combination or in the absence of a heater.

In the embodiment of a mouthpiece portion depicted in Fig. 1, the mouthpiece portion is a atomizer/liquid reservoir portion 14 which further comprises a tempering element 50 connected to connecting elements 54 which are further connected to contact terminals 52. In the embodiment depicted in Fig. 1, the tempering element 50 includes a tempering coil arranged in the central passage 32. The contact terminals 52 arranged adjacent to one port 44 of two ports 44, 36 connected by the central passage 32, the other port 36 of two ports 44, 36 serving as an air inhalation port.

Using the contact terminals 52 which contact counter contact terminals 58 of the battery portion which are connected to the control electronics 22, the tempering element 50 is connected to the control electronics 22 in parallel to the atomizer 26. Through tempering, condensation of the aerosol is prevented or reduced compared to a central passage in which no tempering is provided. The tempering may be such that a temperature drop within the tempered section is avoided or reduced. The tempering may be such that a temperature increase occurs in the tempered section. A temperature of the tempering element when operated may be lower than a threshold temperature at or above which droplets of the aerosol evaporates. Particularly, when operated the tempering element and the heating coil of the atomizer may have significantly different temperatures.

In embodiments of electronic cigarettes where battery portion and atomizer/liquid reservoir portion are formed as a single piece or where battery portion and atomizer/liquid reservoir portion cannot be separated once fitted together the contact counter contact terminals 58 of the battery portion may be formed integrally with the control electronics 22 or with the conductive elements 54. Then, the conductive elements 54 extend from the control electronics 22 to the tempering element 50.

In the example shown, the tempering element 50 is a coil is wrapped around the central passage 32. Other tempering elements may be used. The tempering element 50 may be positioned between in the atomizer 26 and the air inhalation port 36 and may be transverse or parallel to the liquid reservoir 34. The tempering element 50 does not completely block the central passage 32. Rather an air gap is provided on either side of the tempering element 50 enabling air to flow past the tempering element 50.

In the example shown in Figure 1 there are further provided a catalytic converter 62 and a filter 64 in the section of the central passage 32 surrounded by the tempering element 50. However presence of one or both of the catalytic converter 62 and the filter 64 is optional.

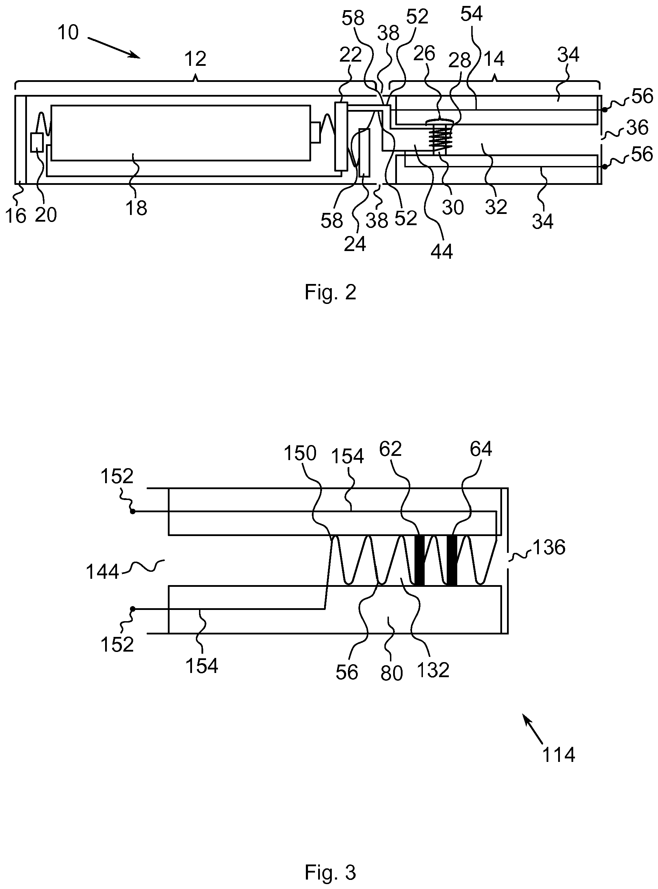

In a further embodiment of a mouthpiece portion depicted in Fig. 2, the mouthpiece portion is an atomizer/liquid reservoir portion 14 which further comprises connecting elements 54 which are connected to contact terminals 52 and which further are connected to further contact terminal contact terminals 56. Using the connecting elements 52 and the contact terminals 54 which contact counter contact terminals 58 of the battery portion which are connected to the control electronics 22, the further contact terminals 56 are connected to the control electronics 22 such that a tempering element can be connected in parallel to the atomizer 26 using the further contact terminals 56.

In embodiments of electronic cigarettes where battery portion and atomizer/liquid reservoir portion are formed as a single piece or where battery portion and atomizer/liquid reservoir portion cannot be separated once fitted together the contact counter contact terminals 58 of the battery portion may be formed integrally with the control electronics 22 or with the conductive elements 54. Then, the conductive elements 54 extend from the control electronics 22 to the further contact terminals 56.

For instance, a further mouthpiece portion (or mouthpiece extension portion) 114 according to yet another embodiment can be connected to the control electronics 22 using the further contact terminals. In this further embodiment, the other port 36 of the atomizer/liquid reservoir portion 14 acts as an aerosol provision port.

In the example of yet a further mouthpiece 114 shown in in Figure 3, the tempering element 50 is a coil arranged in a central passage 132 of the further mouthpiece 114. The central passage 132 of the further mouthpiece 114 extends from one port 144 of two ports 144, 136 of the further mouthpiece 114 to another port 136 of said two ports 144, 136 of the further mouthpiece 114. The tempering element 50 is connected to via connecting elements 154 to contact terminals 152 arranged in an area surrounding the one port 144. The central passage 132 of the further mouthpiece 114 hence provides an extension of the central passage 32 of the mouthpiece 14.

The coil 150 surrounds the central passage 132 in the further mouthpiece 114. In the embodiment depicted it is surrounded by a further element 80. The further element 80 is optional. As well, the further element 80 may be surrounded by the tempering element 50. If present the further element 80 can be one of: a liquid reservoir, a powder reservoir or an isolating element.

When being attached to the mouthpiece of Figure 2 the elements 152 of the further mouthpiece 114 are in electrical contact with the further contact terminals 56 of the mouthpiece 14. The further contact terminals 56 are connectable to a battery 18 wherein control electronics 22 control the connection.

When connected to the battery, the tempering element 50 hence is operable to temper the aerosol provided through the attachment port 144 into the central passage 132 of the further mouthpiece 114. Through tempering, condensation of the aerosol is prevented or reduced compared to a central passage 132 in which no tempering is provided. The tempering may be such that a temperature drop within the tempered section is avoided or reduced. The tempering may be such that a temperature increase occurs in the tempered section. A temperature of the tempering element when operated may be lower than a threshold temperature at or above which condensate of the aerosol evaporates. Particularly, when operated the tempering element and the heating coil of the atomizer may have significantly different temperatures.

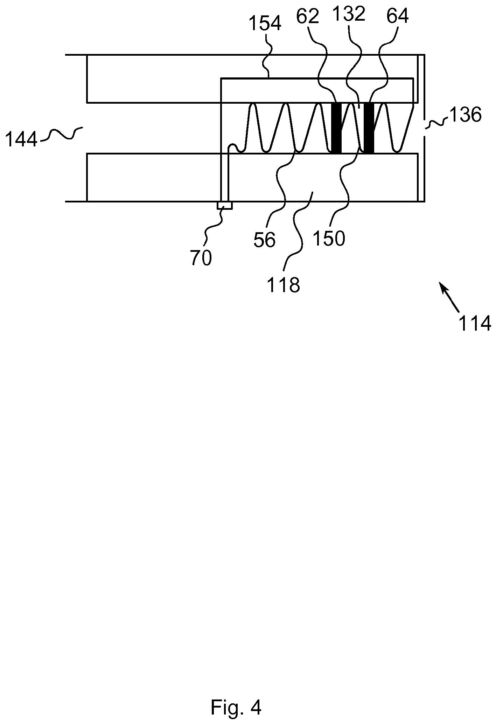

Figure 4 shows even yet another example of the mouthpiece 114 in more detail. The tempering element is a coil 50 connected to a further battery 118 of the further mouthpiece 114. The mouthpiece 114 comprises an input element 70 for a user to activate the tempering element 50 by an input. The input element 70 may a push button. The input element 70 is optional.

When connected to the battery, the tempering element 50 hence is operable to temper the aerosol provided through the attachment port 144 into the central passage 132 of the further mouthpiece 114. Through tempering, condensation of the aerosol is prevented or reduced compared to a central passage in which no tempering is provided. The tempering may be such that a temperature drop within the tempered section is avoided or reduced. The tempering may be such that a temperature increase occurs in the tempered section. A temperature of the tempering element, when operated, may be lower than a threshold temperature at or above which condensate of the aerosol evaporates. Particularly, when operated the tempering element and the heating coil of the atomizer may have significantly different temperatures.

In the examples shown in Figure 3 and 4 there are further provided a catalytic converter 62 and a filter 64 in the section of the central passage 132 surrounded by the tempering element 50. However presence of one or both of the catalytic converter 62 and the filter 64 is optional in both examples.

A mouthpiece according to an embodiment for an electronic smoking device comprises an elongated housing having ports at opposite ends, a central passage formed as a hollow channel through the housing in communication with the ports, contact terminals for connecting to a battery of the electronic smoking device and conductive elements connected to the contact terminals, the electric conductive elements being configured for connecting, to the battery, an tempering element for tempering an aerosol produced by the electronic smoking device.

The mouthpiece can be an atomizer/liquid reservoir portion of an electronic smoking device, wherein the contact terminals are arranged at one of the ports, the atomizer/liquid reservoir portion further comprising an atomizer arranged in the central passage operable when connected to the battery for the electronic smoking device to atomize liquid stored in a liquid reservoir such that the aerosol is provided in the central passage.

The mouthpiece may further comprise the tempering element arranged in the central passage and connected to the conductive elements. The tempering element may extend from the other port at least along a section of the central passage. The tempering element may surround the section of the central passage. The tempering element may surround a further element surrounding the section of the central passage. The mouthpiece may further comprise the liquid reservoir. The mouthpiece may further comprise further contact terminals arranged at the other of the ports for connecting the tempering element. The mouthpiece may further comprise a filter arranged in the central passage.

The mouthpiece may further comprise a catalytic converter arranged in the central passage. The tempering element may be operable when connected to the battery to temper the aerosol such condensation of the aerosol is prevented or reduced. The one port may be configured for attachment to a further mouthpiece having the other port correspondingly configured wherein, by the attachment, the contact terminals get in contact with the further contact terminals of the further mouthpiece. At least one coupling element for releasable attachment of the attachment port to the other port of the further mouthpiece may be comprised by the mouthpiece.

The mouthpiece may further comprise the battery wherein the one port is configured for attachment to an aerosol provision port at one end of a further housing of an atomizer/liquid reservoir portion further comprising a further central passage through the further housing, a liquid reservoir, and an atomizer operable when connected to a further battery for the electronic smoking device to atomize liquid stored in the liquid reservoir such that the aerosol is provided into the central passage from the further central passage through the aerosol provision port and the one port.

In an exemplary embodiment an electronic smocking device comprises the mouthpiece. In one embodiment, the tempering element may be operated while the atomizer is operated. The tempering element may be operated only while the atomizer is operated. Or, operation of the tempering element may be continued for a predetermined time after operation of the atomizer is terminated. In one embodiment, the tempering element may be operated if the atomizer is operated and an additional condition is met. The additional condition may be a temperature measured in the central passage not exceeding a threshold temperature or a user input such as pushing of a button. Or, the tempering element may be operated in response to the user input, independent from whether the atomizer is operated.

An embodiment of an electronic cigarette comprises a tempering element tempering an aerosol produced by the electronic smoking device.

Using the tempering element, aerosol passing from the atomizer to the air inhalation port can be tempered and loss of aerosol due to condensation is reduced.

While this invention has been described in connection with what is presently considered to be practical exemplary embodiments, it is to be understood that the invention is not limited to the disclosed embodiments, but, on the contrary, is intended to cover various modifications and equivalent arrangements included within the scope of the appended claims.

### LIST OF REFERENCE SIGNS

- 10: electronic smoking device
- 12: battery portion
- 14, 114: mouthpiece
- 16: end cap
- 18, 118: battery
- 20: light emitting diode (LED)
- 22: control electronics
- 24: airflow sensor
- 26: atomizer
- 28: heating coil
- 30: wick
- 32, 132: central passage
- 34, 134: liquid reservoir
- 36, 136: air inhalation port/ aerosol provision port
- 38: air inlets
- 44, 144: attachment port
- 50, 150: tempering element
- 52, 152: contact terminals
- 54, 154: connecting elements
- 56: further contact terminals
- 58: counter contact terminals
- 62: filter
- 64: catalytic converter
- 70: input element
- 80: further element

## Claims

1. A mouthpiece (14, 114) for an electronic smoking device (10), the mouthpiece (14, 114) comprising:
an elongated housing having ports at opposite ends;
a central passage (32, 132) formed as a hollow channel through the housing in communication with the ports,
a liquid reservoir (34) and an atomizer (26), the atomizer (26) being arranged in the central passage (32) operable when connected to the battery (18) of the electronic smoking device (10) to atomize liquid stored in the liquid reservoir (34) such that the aerosol is provided in the central passage (32, 132),
a tempering element (50, 150) arranged in the central passage (32, 132) for tempering an aerosol produced by the electronic smoking device (10),
contact terminals (52, 152) arranged at one of the ports (44, 144) for connecting to the battery of the electronic smoking device (10), wherein the tempering element (150) extends from the other port (36, 136) at least along a section of the central passage (32, 132) and
conductive elements (54, 154) connected to the contact terminals (52, 152), the conductive elements (54, 154) being further connected to the tempering element (50, 150),
**characterized in that**
the tempering element (50, 150) surrounds the section of the central passage (32, 132) along the longitudinal axis of the central passage (32, 132).

2. The mouthpiece (14, 114) of claim 1, wherein the tempering element (50, 150) surrounds a further element surrounding the section of the central passage (32, 132).

3. The mouthpiece (14, 114) of any one of the preceding claims wherein the mouthpiece (14, 114) further comprises a filter (64) arranged in the central passage (32, 132).

4. The mouthpiece (14, 114) of any one of the preceding claims, wherein the mouthpiece (14, 114) further comprises a catalytic converter (62) arranged in the central passage (32, 132).

5. An electronic smoking device (10) comprising a mouthpiece (14, 114) according to any one of the preceding claims.

## Patentansprüche

1. Mundstück (14, 114) für eine elektronische Rauchvorrichtung (10), wobei das Mundstück (14, 114) Folgendes umfasst:
ein längliches Gehäuse mit Öffnungen an gegenüberliegenden Enden;
einen mittleren Durchlass (32, 132), der als hohler Kanal durch das Gehäuse in Kommunikation mit den Öffnungen ausgebildet ist,
einen Flüssigkeitsreservoir (34) und einen Verdampfer (26), wobei der Verdampfer (26) in dem mittleren Durchlass (32) angeordnet ist und dazu betreibbar ist, wenn er mit der Batterie (18) der elektronischen Rauchvorrichtung (10) verbunden ist, in dem Flüssigkeitsreservoir (34) gespeicherte Flüssigkeit derart zu verdampfen, dass das Aerosol in dem mittleren Durchlass (32, 132) bereitgestellt wird,
ein Temperierelement (50, 150), das in dem mittleren Durchlass (32, 132) dazu angeordnet ist, ein von der elektronischen Rauchvorrichtung (10) produziertes Aerosol zu temperieren,
Kontaktanschlüsse (52, 152), die an einer der Öffnungen (44, 144) dazu angeordnet sind, sich mit der Batterie der elektronischen Rauchvorrichtung (10) zu verbinden,
wobei das Temperierelement (150) sich zumindest entlang eines Abschnitts des mittleren Durchlasses (32, 132) von der anderen Öffnung (36, 136) erstreckt, und
leitfähige Elemente (54, 154), die mit den Kontaktanschlüssen (52, 152) verbunden sind, wobei die leitfähigen Elemente (54, 154) ferner mit dem Temperierelement (50, 150) verbunden sind,
**dadurch gekennzeichnet, dass**
das Temperierelement (50, 150) den Abschnitt des mittleren Durchlasses (32, 132) entlang der Längsachse des mittleren Durchlasses (32, 132) umgibt.

2. Mundstück (14, 114) nach Anspruch 1, wobei das Temperierelement (50, 150) ein weiteres Element umgibt, das den Abschnitt des mittleren Durchlasses (32, 132) umgibt.

3. Mundstück (14, 114) nach einem der vorangehenden Ansprüche, wobei das Mundstück (14, 114) ferner einen Filter (64) umfasst, der in dem mittleren Durchlass (32, 132) angeordnet ist.

4. Mundstück (14, 114) nach einem der vorangehenden Ansprüche, wobei das Mundstück (14, 114) ferner einen Katalysator (62) umfasst, der in dem mittleren Durchlass (32, 132) angeordnet ist.

5. Elektronische Rauchvorrichtung (10), umfassend ein Mundstück (14, 114) nach einem der vorangehenden Ansprüche.

## Revendications

1. Embout buccal (14, 114) pour un dispositif de cigarette électronique (10), l'embout buccal (14, 114) comprenant :
un boîtier allongé ayant des orifices à des extrémités opposées ;
un passage central (32, 132) formé sous la forme d'un canal creux à travers le boîtier en communication avec les orifices,
un réservoir de liquide (34) et un atomiseur (26), l'atomiseur (26) étant agencé dans le passage central (32) et pouvant être utilisé lorsqu'il est relié à la batterie (18) du dispositif de cigarette électronique (10) pour pulvériser le liquide stocké dans le réservoir de liquide (34) de telle sorte que l'aérosol est fourni dans le passage central (32, 132),
un élément de conditionnement en température (50, 150) agencé dans le passage central (32, 132) pour conditionner un aérosol produit par le dispositif de cigarette électronique (10),
des bornes de contact (52, 152) agencées sur l'un des orifices (44, 144) pour la liaison à la batterie du dispositif de cigarette électronique (10), l'élément de conditionnement en température (150) s'étendant à partir de l'autre orifice (36, 136) au moins le long d'une section du passage central (32, 132), et
des éléments conducteurs (54, 154) reliés aux bornes de contact (52, 152), les éléments conducteurs (54, 154) étant en outre reliés à l'élément de conditionnement en température (50, 150),
**caractérisé en ce que**
l'élément de conditionnement en température (50, 150) entoure la section du passage central (32, 132) le long de l'axe longitudinal du passage central (32, 132).

2. Embout buccal (14, 114) selon la revendication 1, dans lequel l'élément de conditionnement en température (50, 150) entoure un autre élément entourant la section du passage central (32, 132).

3. Embout buccal (14, 114) selon l'une quelconque des revendications précédentes, selon lequel l'embout buccal (14, 114) comprend en outre un filtre (64) agencé dans le passage central (32, 132).

4. Embout buccal (14, 114) selon l'une quelconque des revendications précédentes, dans lequel l'embout buccal (14, 114) comprend en outre un convertisseur catalytique (62) agencé dans le passage central (32, 132).

5. Dispositif de cigarette électronique (10) comprenant un embout buccal (14, 114) selon l'une quelconque des revendications précédentes.
